# NEUE EUROPÄISCHE PATENTSCHRIFT

(11) **EP 0 047 899 B2**
(45) Veröffentlichungstag und Bekanntmachung des Hinweises auf die Entscheidung über den Einspruch: **28.02.1996**
(45) Hinweis auf die Patenterteilung: 04.01.1984
(21) Anmeldenummer: 81106729.7
(22) Anmeldetag: 28.08.1981
(51) Int. Cl.: A61K 9/14, A61K 31/44

(54) **Feste Arzneizubereitungen enthaltend Nifedipin und Verfahren zu ihrer Herstellung**
Solid pharmaceutical compositions containing nifedipine, and process for their preparation
Compositions pharmaceutiques solides contenant de la nifédipine et procédé pour leur préparation

(30) Priorität: 09.09.1980 DE 3033919
(43) Veröffentlichungstag der Anmeldung: 24.03.1982
(73) Patentinhaber: BAYER AG, D-51368 Leverkusen (DE)
(72) Erfinder: Hegasy, Ahmed, Dr., D-5090 Leverkusen 1 (DE); Rämsch, Klaus-Dieter, Dr., Heerlen (NL)

(56) Entgegenhaltungen:
- EP-A- 0 001 247
- EP-B- 0 047 899
- DE-A- 2 348 334
- DE-A- 2 400 819
- DE-A- 2 822 882
- DE-B- 1 492 055
- DE-B- 1 670 827
- DE-C- 2 209 526
- FR-A- 2 256 765
- GB-A- 1 173 862
- GB-A- 1 456 618
- GB-A- 2 053 681
- US-A- 3 784 684
- Pharmacol. Therap. (1984), pp. 742-749
- CHEMICAL ABSTRACTS, Band 87, Nr. 18, 31. Oktober 1977, Seite 323, Nr. 141227n Columbus, Ohio, U.S.A. A.I. TENTSOVA et al.: "Effect of the particle size distribution of drugs on their bioavailability"
- "Angewandte Biopharmazie", 1973, Seiten 293-302
- Pharmacol. Therap. (1984), S. 742-49
- 2. internationales Adalat-Symposium, S. 27-32, 94
- Arzneimittelverzeichnis der DDR, S. 90
- Inn. Med , 1977, Bd. 33, S. 44
- ADALAT Ärzteprospekt, Feb. 1986
- Med. Journal of AUstralia, 1987, Bd. 146, S. 228-29
- Journal of Pharmaceutical Sciences, 1968, Bd. 57, nr. 11
- The Merck Index, 10th ed., Deckblatt u. S. 936-37
- Devel. & Indust. Pharmarcy, 1980, 6, nr. 2
- DIN 66132-Normblatt
- Pharmaceutica Acta Helvetiae, 1. Mitt., Bd. 44 (1969), S. 65-78; 2. Mitt., Bd. 44 (1969) S. 238-256; 3. Mitt. Bd. 45 (1979), S. 461-479
- W.A. Ritschel, Angewandte Biopharmazie, 1973, S. 293-302
- Nature, 10.02.1962, S. 588-89
- Pharm. Weekblad, 1972, 107, S. 485-88
- Lehrbuch der pharmazeutischen Technologie, 1976, S. 692-94
- Medicamentum Bd. 2, 1977, S. 51 und Bd. 6, 1977, S. 185
- Inn. Med. Bd. 32, 1977, S. 479-483
- Medicamentum, 1978, S. 258-261
- Arzneimittelverzeichnis - VEB- 1979, Teil 1, S.90
- J. Pharmaceutical Sciences Bd. 57, 1968, nr. 11, S. 1825-1835
- Acta Pharm. Jugoslav Bd. 27, 1977, S. 7-11
- Pharmazie, Bd. 34, 1979, S. 242-43
- J. Pharm. Sci. Bd. 68, 1979, nr. 7, S. 850-852
- J. Pharm. Sci. Band 56, 1967, nr. 7, S. 882-85

## Beschreibung

Die vorliegende Erfindung betrifft die Verwendung von Nifedipinkristallen mit einer spezifischen Oberfläche von 1 - 4 m²/g zur Herstellung von festen Arzneizubereitungen mit langer Wirkungsdauer.

Es ist bereits bekannt geworden, dass die Verbindung Nifedipin sehr starke kreislaufbeeinflussende Wirkungen besitzt (vgl. britisches Patent 1 173 862) Aufgrund der Lichtempfindlichkeit und der schweren Löslichkeit von Nifedipin treten bei der galenischen Zubereitung von Arzneispezialitäten eine Reihe von Schwierigkeiten auf, wie aus zahleichen Patenten und Patentanmeldungen für spezielle Formulierungen dieses Wirkstoffs ersichtlich wird. So betrifft z.B. das US-Patent 3 784 684 spezielle Nifedipin enthaltende Gelatinebeisskapseln, durch welche die Coronarwirkung von Nifedipin vorteilhaft genutzt werden kann. In dem britischen Patent 1 456 618 werden weiterhin feste Arzneizubereitungen beschrieben und beansprucht, welche ebenfalls eine gute Bioverfügbarkeit von Nifedipin gewährleistet. Auch in der DT-OS 2 822 882 werden feste Arzneiformen beschrieben, bei denen durch den Einsatz bestimmter Losungsvermittler und oberflachenaktiver Substanzen die Schwerlöslichkeit von Nifedipin kompensiert werden soll. Auch in der europäischen Offenlegungsschrift 1 247 soll die Resorbierbarkeit von Nifedipin durch die Verwendung von Polyethylenglykol und bestimmter poröser Trägersubstanzen verbessert werden.

Alle bisherigen Versuche, die schlechte Löslichkeit von Nifedipin durch bestimmte Massnahmen zu kompensieren und gleichzeitig eine gute Bioverfügbarkeit zu gewährleisten, besitzen eine Reihe von Nachteilen. Der Einsatz von oberflächenaktiven Substanzen, Lösungsvermittlern und bestimmten Trägerstoffen, die eine besondere Oberfläche haben, z.B. porös sind, führt häufig zu Verabreichungsformen bei denen die Präparate unerwünscht gross sind Zur Erleichterung des Schluckens werden solche Tabletten oder Kapseln häufig in spezifische Formen wie z.B. Elypsoide oder Längsformen überführt, was jedoch bei Präparaten mit einem Gewicht über 400 mg auch nicht mehr zu befriedigenden Ergebnissen führt. Auch das häufigere Einnehmen von kleineren Präparaten stellt keine befriedigende Lösung dar.

Für Arzneizubereitungen gilt, dass sowohl die Zahl als auch die Menge der Hilfs- und Träger, stoffe möglichst niedrig gehalten werden soll. Beim Vergleich zweier Arzneispezialitäten wird man immer demjenigen Präparat den Vorzug geben, welches neben dem Wirkstoff möglichst wenig Hilfsstoffe und Zuschlagstoffe enthält, um unerwünschte biologische Wirkungen weitgehend zu vermeiden.

Ein weiterer Nachteil der bisher bekannten Nifedipin enthaltenden Präparate liegt in dem aufwendigen Herstellungsverfahren, welches insbesondere für flüssige Zubereitungen und Kapselpräparate gilt Die hohe Lichtempfindlichkeit und die schwere Löslichkeit von Nifedipin bedingen aufwendige Verfahrensmassnahmen, die insbesondere bei Flüssigzubereitungen als Lichtschutz, den Ausschluss von Tageslicht und die Verwendung von Natriumlicht erfordern.

Die vorliegende Erfindung betrifft die Verwendung von Nifedipinkristallen mit einer spezifischen Oberfläche von 1 bis 4 m²/g zur Herstellung von festen Arzneizubereitungen zur Erreichung von lang andauernden Blutspiegeln zur oralen Behandlung von Hypertonie durch 1 bis 2 mal tägliche Applikation.

Die Herstellung der erfindungsgemäßen Arzneizubereitungen erfolgt nach üblichen Methoden, indem man Nifedipin-Kristalle der angegebenen spezifischen Oberfläche mit geeigneten Hilfsstoffen mischt oder granuliert und aus den Mischungen oder Granulaten nach üblichen Verfahren feste Arzneizubereitungen herstellt.

Als feste Arzneizubereitungen seien vorzugsweise genannt: Tabletten, Pillen, Dragees, Kapseln und Sachets.

Die Herstellung der erfindungsgemäß verwendeten Nifedipinkristalle mit der spezifischen Oberfläche von 1 bis 4 m²/g erfolgt, indem man die aus der Synthese von Nifedipin erhaltenen Kristallgemische mahlt. Die Mahlung kann z. B. mit Stiftmühlen oder Hammermühlen erfolgen. Durch Variation der Drehzahl der Mühle, der zulaufenden Menge Produkt und/oder der Mahlungsdauer kann Nifedipin mit der gewünschten Oberfläche gewonnen werden.

Wenn ein Produkt mit einer grösseren spezifischen Oberfläche gewünscht ist, ist es vorteilhaft, die Mahlung mit Luftstrahlmühlen durchzuführen.

Die spezifische Oberfläche wird bestimmt nach der Gasadsorptionsmethode (BET-Methode; vergleiche S. Brunauer: The Adsorption of Gases and Vapours, Princeton (1945)).

Überraschenderweise zeigen die erfindungsgemäßen festen Zubereitungen eine unerwartet hohe Bioverfügbarkeit. In der Publikation von I. Sugimoto et al., Drug Development and Industrial Pharmacy, 6 (2), 137-160 (1980) wird ausdrücklich betont (S. 139), daß Nifedipin in kristalliner Form bei oraler Applikation schlecht absorbiert wird und nur eine sehr geringe Bioverfügbarkeit besitzt. Es konnte daher nicht erwartet werden, daß nach oraler Applikation der erfindungsgemäßen Zubereitung, die kristallines Nifedipin enthält, die Plasmakonzentration schnell ansteigt und sich für viele Stunden auf einem hohen Wert hält. In Fällen, in denen Nifedipin über längere Zeiträume eingenommen werden muß, ist es aufgrund dieser sehr hohen Wirkungsdauer ausreichend, wenn täglich 1 oder 2 Tabletten appliziert werden Ein weiterer wesentlicher Vorteil besteht darin, dass sehr kleine Tabletten mit hohem Wirkstoffgehalt hergestellt werden können, da auf Lösungsvermittler, oberflächenaktive Stoffe und zusätzliche Hilfsstoffe weitgehend verzichtet werden kann.

Die Kleinheit der Tablette und die überraschend grosse Wirkungsdauer der erfindungsgemässen Formulierung ermöglichen es, die blutdrucksenkende Wirkung von Nifedipin zur Behandlung von Hypertonie einzusetzen, Die durch die erfindungsgemässe Formulierung erhältlichen lang andauernden Blutspiegel des Wirkstoffs stellen eine Erweiterung der praktischen Anwendungsmöglichkeiten für Nifedipin dar und bedeuten gleichzeitig eine Erleichterung für den Patienten.

Die folgenden Ausführungsbeispiele erläutern die Erfindung.

### Beispiel 1

200 g von Nifedipinkristallen mit einer spezifischen Oberfläche von 4 m²/g werden mit 348 g mikrokristalliner Cellulose, 100 g Lactose, 10 g Tween^{R} 80, 70 g Stärke und 2 g Magnesiumstearat vermischt. Aus weiteren 70 g Stärke wird mit Wasser in an sich bekannter Weise ein Kleister hergestellt, der mit der o.g. Mischung üblicherweise granuliert, getrocknet und anschliessend zu Tabletten mit einem Einzelgewicht von 80 mg gepresst werden. Anschliessend werden diese Tabletten markiert, sie besitzen einen Druchmesser von 6 mm.

Zur Lackierung von g 800 Tabletten wird z.B. eine Suspension aus
18 g Hydroxypropylmethylcellulose,
6 g Polyethylenglykol
5,4 g Titandioxid
0.6 g Eisenoxid und
370 g Wasser oder Ethanol benutzt.

Bei Kenntnis des Standes der Technik, aus welchem ersichtlich ist, dass die Fachwelt seit Jahren bemüht ist, brauchbare Zubereitungsformen für den schwer zu formulierenden Wirkstoff Nifedipin zu finden, ist es als ausgesprochen überraschend zu bezeichnen, dass durch die Auswahl einer ganz bestimmten spezifischen Oberfläche des Wirkstoffs ein sehr einfaches und wirkungsvolles Prinzip zur galenischen Verarbeitung gefunden wurde.

Die erfindungsgemässen festen Zubereitungsformen stellen für den Patienten eine Erleichterung bei der Applikation dar und erhöhen gleichzeitig die Sicherung seiner Behandlung.

Zum Nachweis der vorteilhaften Wirkung der erfindungsgemässen Arzneizubereitungen wurde bei jeweils 8 Personen über mehrere Stunden nach der Applikation die Plasmakonzentration ermittelt. Die Werte sind aus der folgenden Tabelle ersichtlich:

**Tabelle**

| Zeit (Stunden) | 1 | 2 | 3 | 4 | 6 | 8 | 10 | 25,5 |
|---|---|---|---|---|---|---|---|---|
| Plasmakonz. µg/l nach p.o. Applikation der Tabletten aus Beispiel 1 (20 mg) | 105,8 | 86,1 | 65,3 | 63,9 | 43,1 | 46,7 | 11,8 | 10,8 |
| dto. nach p.o. Applikation der Tabletten aus Beispiel 2 (20 mg) | 52,1 | 66,3 | 60,4 | 51,3 | 32,4 | 25 | 18,8 | 11,4 |

### Beispiel 2

200 g Nifedipinkristalle mit einer spezifischen Oberfläche von 1 m²/g werden analog Beipsiel 1 zu 80 mg Tabletten mit einem Durchmesser von 6 mm gepresst.

### Beispiel 3

200 g Nifedipin mit einer spezifischen Oberfläche von 1,2 m²/g werden mit 800 g Lactose, 960 g Stärke und 40 g Magnesiumstearat gemischt. Die Mischung wird in Hartgelatinekapseln der Grösse 3 zu 100 mg Mischung abgefüllt. Pro Kapsel sind dann 10 mg Nifedipin enthalten. Durch Variation der Kapselgrösse und Inhaltsgewichte können Kapseln mit verschiedenen Dosierungen hergestellt werden, z.B. zwischen 5 mg und 40 mg Wirkstoff pro Kapsel.

## Patentansprüche

1. Verwendung von Nifedipinkristallen mit einer spezifischen Oberfläche von 1 - 4 m²/g zur Herstellung von festen Arzneizubereitungen zur Erreichung von lang andauernden Blutspiegeln zur oralen Behandlung von Hypertonie durch 1 bis 2 mal tägliche Applikation.

## Claims

1. Use of nifedipine crystals with a specific surface area of 1-4 m²/g for the production of solid medicinal formulations for achieving long-lasting blood levels for the oral treatment of hypertension by administration 1 to 2 times a day.

## Revendications

1. Utilisation de cristaux de nifédipine ayant une surface spécifique de 1 à 4 m²/g pour la production de préparations pharmaceutiques solides permettant d'obtenir des taux sanguins de durée prolongée en vue du traitement oral de l'hypertomie par administration une à deux fois par jour.
